# EUROPÄISCHE PATENTSCHRIFT

(11) **EP 3 024 522 B1**
(45) Veröffentlichungstag und Bekanntmachung des Hinweises auf die Patenterteilung: **26.09.2018**
(21) Anmeldenummer: 14741249.8
(22) Anmeldetag: 11.07.2014
(51) Int. Cl.: A61M 15/00

(54) **GEHÄUSE FÜR EINE INHALATIONSVORRICHTUNG UND INHALATIONSVORRICHTUNG ZUR ORALEN VERABREICHUNG EINES PHARMAZEUTISCHEN MEDIUMS**
HOUSING FOR AN INHALATION DEVICE AND INHALATION DEVICE FOR ORALLY ADMINISTERING A PHARMACEUTICAL MEDIUM
BOÎTIER D'UN DISPOSITIF D'INHALATION ET DISPOSITIF D'INHALATION POUR L'ADMINISTRATION ORALE D'UN AGENT PHARMACEUTIQUE

(30) Priorität: 25.07.2013 DE 102013214601
(43) Veröffentlichungstag der Anmeldung: 01.06.2016
(73) Patentinhaber: Aptar Radolfzell GmbH, 78315 Radolfzell (DE)
(72) Erfinder: KOHNLE, Jörg, 78056 Villingen-Schwenningen (DE); KEPPNER, Frank, 72458 Albstadt (DE)
(74) Vertreter: Patentanwaltskanzlei Cartagena
(86) Internationale Anmeldenummer: PCT/EP2014/064978
(87) Internationale Veröffentlichungsnummer: WO 2015/010932

(56) Entgegenhaltungen:
- GB-A- 2 470 188
- US-A- 6 138 669

## Beschreibung

Die Erfindung betrifft ein Gehäuse für eine Inhalationsvorrichtung zur oralen Verabreichung eines pharmazeutischen Mediums und eine Inhalationsvorrichtung. Eine gattungsgemäße Inhalationsvorrichtung umfasst eine Containereinheit mit einem Mediumcontainer und einem Auslassstutzen, wobei zum Zwecke eines Austragvorgangs der Auslassstutzen relativ zu dem Mediumcontainer verlagerbar ist. Das Gehäuse für eine derartige Inhalationsvorrichtung umfasst ein Grundgehäuse mit einem ersten, ein Mundstück bildenden Ende, einem zweiten, zu dem ersten Ende L-förmig abgewinkelten, einen Lufteinlass bildenden Ende und einem Anschlussstück, wobei das Anschlussstück einen Kanal mit einer Anschlussöffnung zur Aufnahme des Auslassstutzens der Containereinheit, eine in Richtung des ersten Endes mündenden Düsenöffnung und einer zwischen der Anschlussöffnung und der Düsenöffnung angeordnete Druckkammer aufweist. Das gattungsgemäße Gehäuse umfasst weiter einen Taster zur Erfassung eines Austragvorgangs.

Gattungsgemäße Inhalationsvorrichtungen werden üblicherweise als "MDI" ("Metered-Dose Inhaler") oder "pMDI" ("pressurized Metered-Dose Inhaler") bezeichnet. Sie dienen der Verabreichung von Medikamenten, die zum Zwecke der Behandlung von Atemwegserkrankungen in zerstäubter Form in die Lunge des Benutzers gelangen sollen. Sie weisen das genannte Gehäuse auf, in welches die genannte Containereinheit eingesetzt ist. Das Gehäuse verfügt über einen Lufteinlass, der es gestattet, Luft zu dem Mundstück hin einzusaugen, wobei zeitgleich mit dem Einsaugen der Austragvorgang von Medium aus dem Mediumcontainer durch Verlagern des Mediumcontainers gegenüber dem Auslassstutzen bewirkt wird, sodass die eingesogene Luft mit dem zerstäubten Medium vermengt und eingeatmet wird und somit in die Lunge gelangen kann.

Der Austrag von Medium aus dem Mediumcontainer wird durch die Relativverlagerung des Mediumcontainers gegenüber dem Auslassstutzen bewirkt. Im einfachsten Falle kann bei gattungsgemäßen Inhalationsvorrichtungen der Mediumcontainer hierzu ein Stück weit aus dem Lufteinlass des Gehäuses herausragen, sodass er unmittelbar manuell kraftbeaufschlagt werden kann, um gegenüber dem in eine korrespondierende Ausnehmung des Gehäuses eingesetzten Auslassstutzen verlagert zu werden.

Um einem Benutzer der Inhalationseinrichtung die Möglichkeit zu geben, schnell und sicher die Zahl der noch in dem Mediumcontainer verbleibenden Dosen zu erfassen, sind sogenannte Austragsensoren zum Erfassen einzelner Austragvorgänge bekannt.

Zu diesem Zweck ist es beispielsweise aus DE 10 2010 024 912 A1 bekannt, ein Gehäuse vorzusehen, dass einen Hauptabschnitt und einen gegenüber dem Hauptabschnitt verlagerbaren Sensorwandungsabschnitt aufweist, wobei der Sensorwandungsabschnitt derart ausgebildet und/oder angeordnet ist, dass er während eines Austragvorgangs gegenüber dem Hauptabschnitt verlagert wird, und wobei die Verlagerung durch einen Taster erfassbar ist. Als Taster wird im Zusammenhang mit der Anmeldung ein Bedienelement bezeichnet, welches durch Drücken betätigt wird und nach einer Betätigung in die Ausgangslage zurückkehrt. Für die Rückführung in die Ausgangslange wird in vorteilhaften Ausgestaltungen eine mechanische Feder eingesetzt. Die Verwendung eines Tasters ist aufgrund der Zuverlässigkeit sowie der niedrigen Herstellungskosten besonders vorteilhaft. Die aus DE 10 2010 024 912 A1 bekannte Gestaltung zeichnet sich somit durch die einfache und damit zuverlässige und kostengünstige Bauweise aus.

Alternativ ist beispielsweise aus WO 1997/033640 A1 bekannt, einen Drucksensor an einem für den Medienaustrag vorgesehenen Kanal anzuordnen ist und den Mediumaustrag dadurch erfassbar macht. Diese Gestaltung ist vergleichsweise komplex und teuer.

Aus der GB 2470188 A und der US 6138669 A sind Inhalationsvorrichtungen bekannt bekannt, bei denen vorgesehen ist, dass eine Auswertung der Betätigung durch einen Benutzer auf Basis eines Druckanstiegs in einer Druckkammer erfolgt. Hierfür ist in der Druckkammer eine Membran bzw. ein Gelkörper angeordnet, die bzw. der durch den Druck auslenkbar ist und hierdurch einen Kontakt zum Zwecke des Zählens schließen kann.

### Aufgabe und Lösung

Aufgabe der Erfindung ist es, ein Gehäuse für eine Inhalationsvorrichtung und eine Inhalationsvorrichtung zu schaffen, mittels welcher zuverlässig die Austragvorgänge erfassbar sind. Dabei soll insbesondere auch eine kostengünstige Gestaltung erreicht werden.

Gemäß einem ersten Aspekt wird ein Gehäuse für eine Inhalationsvorrichtung zur oralen Verabreichung eines pharmazeutischen Mediums geschaffen, umfassend ein Grundgehäuse mit einem ersten, ein Mundstück bildenden Ende, einem zweiten, zu dem ersten Ende L-förmig abgewinkelten, einen Lufteinlass bildenden Ende und einem Anschlussstück, welches einen Kanal mit einer Anschlussöffnung zur Aufnahme des Auslassstutzens der Containereinheit, mit einer in dem Mundstück mündenden Düsenöffnung und mit einer zwischen der Anschlussöffnung und der Düsenöffnung angeordnete Druckkammer aufweist, einen Taster zur Erfassung eines Austragvorgangs und eine in Abhängigkeit eines Drucks in der Druckkammer reversibel verformbare Membran, welche mit dem Taster für dessen Betätigung zusammenwirkt.

Gemäß einem zweiten Aspekt wird eine Inhalationsvorrichtung zur oralen Verabreichung eines pharmazeutischen Mediums geschaffen, umfassend eine Containereinheit mit einem Mediumcontainer und einem Auslassstutzen und ein Gehäuse, wobei das Gehäuse einen Taster zur Erfassung eines Austragvorgangs und ein Grundgehäuse mit einem ersten, ein Mundstück bildenden Ende, einem zweiten, zu dem ersten Ende L-förmig abgewinkelten, einen Lufteinlass bildenden Ende und einem Anschlussstück aufweist, wobei das Anschlussstück einen Kanal mit einer Anschlussöffnung zur Aufnahme des Auslassstutzens der Containereinheit, mit einer in dem Mundstück mündenden Düsenöffnung und mit einer zwischen der Anschlussöffnung und der Düsenöffnung angeordnete Druckkammer aufweist, und wobei eine in Abhängigkeit eines Drucks in der Druckkammer reversibel verformbare Membran vorgesehen ist, welche mit dem Taster für dessen Betätigung zusammenwirkt.

Durch eine Verlagerung des Mediumcontainers relativ zu dem Auslassstutzen und damit relativ zu dem Anschlussstück wird ein Sprühstoß oder kurz Sprüh abgegeben. Hierfür wird Medium von der Containereinheit in die Druckkammer abgegeben, welches nachfolgend über die Düsenöffnung in Richtung Mundstück ausgetragen wird. Aufgrund des Mediums in der Druckkammer steigt ein Druck in der Druckkammer und die Membran wird verformt. Die verformte Membran übt auf einen Stempel des Tasters ein Druck aus. Ein Austragvorgang wird dabei unmittelbar basierend auf einem mit dem Austragvorgang einhergehenden Druckanstieg in der Druckkammer erfasst.

Die Membran ist aus einem elastisch verformbaren Material gefertigt, welches durch den Fachmann entsprechend eines für die Inhalationsvorrichtung spezifizierten Druck- und/oder Temperaturbereich geeignet gewählt ist. Geeignete Materialien sind beispielsweise Silikon, natürliche oder synthetische Polymerdispersionen, thermoplastische Elastomere oder Kombinationen davon.

Erfindungsgemäß ist vorgesehen, dass eine mit der Druckkammer kommunizierte, durch die verformbare Membran gegenüber der Umgebung abgeschlossene Messkammer vorgesehen ist. Dadurch ist es möglich, den Taster, welcher durch die sich verformende Membran betätigt wird, räumlich getrennt zu der Druckkammer vorzusehen. Die Membran kann als gegenüber den übrigen Gehäusebestandteilen separates Bauteil ausgebildet sein, welches im Zuge der Montage mit diesen zusammengefügt wird. Besonders vorteilhaft ist jedoch eine Gestaltung, bei der die Membran einerseits und umgebende Gehäuseteile, die aus einem anderen und insbesondere starreren Material bestehen, in einem gemeinsamen Fertigungsprozess durch Mehrkomponentenspritzguss hergestellt sind. Die Membran ist dadurch stoffschlüssig mit den umgebenden Gehäuseteile verbunden.

Erfindungsgemäß ist vorgesehen, dass die Messkammer mit der Druckkammer über eine Bohrung kommuniziert ist, wobei vorzugsweise ein Durchmesser der Bohrung zwischen ca. 120% und ca. 500% des Durchmessers der Düsenöffnung beträgt. Ein Durchmesser der Bohrung ist dabei auch abhängig von einer Größe der Druckkammer. Vorzugsweise beträgt ein Durchmesser der Bohrung maximal 50% des Durchmessers der Druckkammer. Das Anschlussstück ragt in typischen Ausgestaltungen von einer Bodenfläche des Grundgehäuses ab, wobei eine Erstreckungsrichtung des Anschlussstücks parallel zu einer Erstreckungsrichtung des Grundgehäuses in Richtung des zweiten Endes ist. Die Bohrung erstreckt sich in einer Ausgestaltung parallel zur Erstreckungsrichtung des Anschlussstücks ausgehend von der Druckkammer in Richtung der Bodenfläche des Grundgehäuses. Für eine einfache Herstellung sind die Ausnehmung, die Druckkammer und eine die Druckkammer und die Messkammer verbindende Bohrung dabei fluchtend zu einander angeordnet.

In bevorzugten Ausgestaltungen zweigt die Bohrung quer, vorzugsweise im Wesentlichen senkrecht zu einer Sprührichtung der Containereinheit in Sprührichtung stromaufwärts der Düsenöffnung von der Druckkammer ab. Die Düsenöffnung und die Bohrung sind dabei vorzugsweise um ca. 180° zueinander versetzt angeordnet. Dadurch wird erreicht, dass auf die Membran ein Druck aufgrund des abgegebenen Mediums wirkt, durch welchen die Membran ausgelenkt wird. Eine Impulskraft aufgrund eines Sprühs bewirkt dabei nur eine geringe oder keine Veränderung der Auslenkung aufgrund des beschriebenen, von dem Medium bewirkten Druckanstiegs. Durch diese Ausgestaltung ist es zuverlässig möglich, einen vollständigen Sprüh von einem nicht vollständigen Sprüh, beispielsweise aufgrund einer Fehlbedienung, zu unterscheiden.

In einer weiteren Ausgestaltung ist vorgesehen, dass in dem Anschlussstück eine Ausnehmung zur Bildung der Messkammer vorgesehen ist. Die Ausnehmung zur Bildung der Messkammer ist dabei in einer Ausgestaltung angrenzend an eine Mantelfläche an dem Anschlussstück ausgebildet.

In einer Ausgestaltung ist das Grundgehäuse mit der Membran als gemeinsames Spritzgussteil gefertigt, wobei in einer Ausgestaltung die Membran als Einlageelement bei Spritzen des Gehäuses eingebracht wird. In vorteilhaften Ausgestaltungen ist in die Ausnehmung eine die Membran aufweisende Haltevorrichtung eingesetzt. Die Haltevorrichtung ist derart gestaltet, dass sie ein Ausdehnen der Membran für eine Betätigung des Tasters gestattet. Vorzugsweise ist die Haltevorrichtung derart gestaltet, dass sie ein zentrisches Ausdehnen der Membran erlaubt. Die Form oder Gestalt der Haltevorrichtung ist an eine Form oder Gestalt der Ausnehmung angepasst. In vorteilhaften Ausgestaltungen ist eine kreiszylindrische Ausnehmung vorgesehen, in welche eine Haltevorrichtung mit einem kreisringförmigen Querschnitt einsetzbar ist. Eine derartige Haltevorrichtung ist auf einfache Weise in dem Grundgehäuse montierbar.

Die Membran ist in einer Ausgestaltung als Einlegeelement in der Haltevorrichtung angeordnet. In vorteilhaften Ausgestaltungen sind die Membran und die Haltevorrichtung als einstückiges Bauteil gefertigt.

In einer weiteren Ausgestaltung ist der Taster an der Haltevorrichtung gelagert. Der Taster, die Membran und die Haltevorrichtung bilden so eine gemeinsame Baueinheit, welche an dem Grundgehäuse montierbar ist.

Die Inhalationsvorrichtung ist in der Regel in einer aufrechten Position zu betätigen. Wird die Inhalationsvorrichtung in einer davon abweichenden Ausrichtung betätigt, so wird in vielen Fällen eine Dosierkammer des Mediumcontainers nicht korrekt gefüllt und der darauf folgende Sprüh ist nicht vollständig. Die Membran ist vorzugsweise derart ausgelegt und/oder angeordnet, dass ein nicht vollständiger Sprüh eine andere Auslenkung der Membran bewirkt als ein vollständiger Sprüh. Gemäß einer ersten Ausgestaltung bewirkt nur ein vollständiger Sprüh eine Betätigung des Tasters. In vorteilhaften Ausgestaltungen ist der Taster ausgelegt und/oder angeordnet, um zu erfassen, wie stark und/oder wie schnell eine Betätigung erfolgte. Zu diesem Zweck umfasst der Taster in einer Ausgestaltung zwei hintereinander angeordnete Betätigungselemente. Die Gestaltung ermöglicht es, dass zwischen einem vollständigen Sprüh und einer nicht korrekten Ausbringung unterschieden werden kann, wobei beide Ausbringungsvorgänge erfasst werden.

Das Gehäuse ist in vorteilhaften Ausgestaltungen mehrteilig ausgebildet, umfassend das Grundgehäuse und einen an dem Grundgehäuse anbringbaren, elektronische Komponenten einer Zähleinheit einhausenden Verschlussdeckel. Die elektronischen Komponenten sind dabei in einem getrennten Raum vor einem Zugriff und/oder einem Kontakt mit dem Medium geschützt angeordnet.

### Kurzbeschreibung der Zeichnungen

Weitere Vorteile und Aspekte der Erfindung ergeben sich außer aus den Ansprüchen auch aus der nachfolgenden Beschreibung eines Ausführungsbeispiels der Erfindung, das nachfolgend anhand der Figuren erläutert ist. Dabei zeigen:
- Fig. 1: schematisch eine Inhalationsvorrichtung umfassend eine Containereinheit und ein Gehäuse in einer teilweise freigeschnittenen Seitenansicht und
- Fig. 2: schematisch ein Detail des Gehäuses der Inhalationsvorrichtung gemäß Fig. 1 in einer Schnittdarstellung.

### Detaillierte Beschreibung der Ausführungsbeispiele

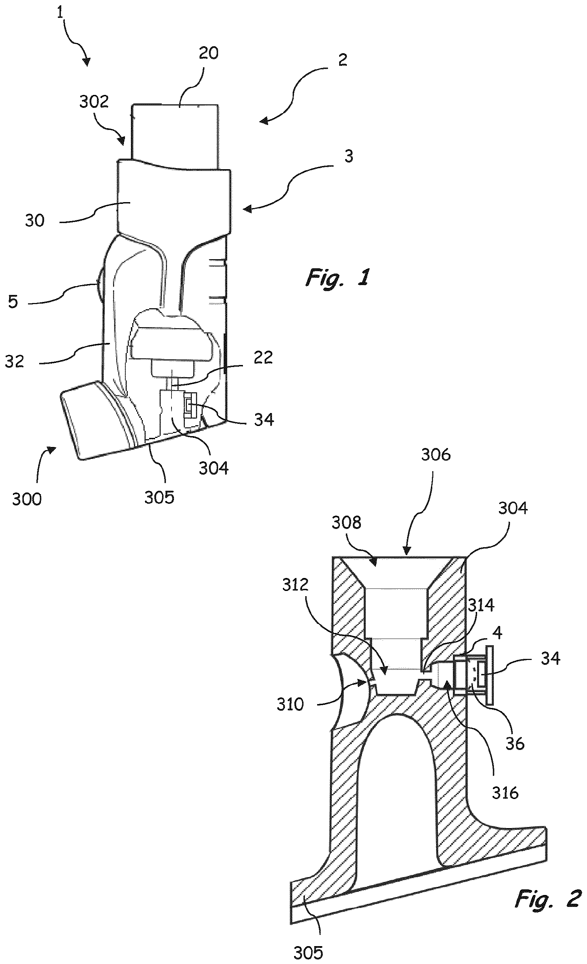

Figur 1 zeigt schematisch eine Inhalationsvorrichtung 1 in einer teilweise freigeschnittenen Seitenansicht. Fig. 2 zeigt ein Detail gemäß Fig. 1 in einer geschnittenen Darstellung.

Die Inhalationsvorrichtung 1 umfasst eine Containereinheit 2 mit einem Mediumcontainer 20 und einem Auslassstutzen 22. Die Inhalationsvorrichtung 1 umfasst weiter ein Gehäuse 3 mit einem Grundgehäuse 30, einem Verschlussdeckel 32 und einem Taster 34.

Das Grundgehäuse weist ein erstes, ein Mundstück bildendes Ende 300 und ein zweites, zu dem ersten Ende 300 L-förmig abgewinkeltes, einen Lufteinlass bildendes Ende 302 auf. Das Grundgehäuse 30 umfasst weiter ein Anschlussstück 304, welches von einer Bodenfläche 305 des Grundgehäuses 30 in Richtung des zweiten Endes 302 abragt.

Wie in Fig. 2 erkennbar ist, weist das Anschlussstück 304 einen Kanal 306 mit einer Anschlussöffnung 308, einer in Richtung des ersten Endes 300 des Grundgehäuses 30 mündenden Düsenöffnung 310 und einer zwischen der Anschlussöffnung 308 und der Düsenöffnung 310 angeordnete Druckkammer 312 auf. Die Anschlussöffnung 308 dient der Aufnahme des Auslassstutzens 22 der Containereinheit 2 gemäß Fig. 1. Die Anschlussöffnung 308 und der Auslassstutzen 22 sind dabei aneinander angepasst, sodass eine klemmende Aufnahme des Auslassstutzens 22 in dem Anschlussstück 304 möglich ist. Die Darstellung sämtlicher Elemente, insbesondere auch der Druckkammer 312, ist in Fig. 2 lediglich schematisch.

In dem dargestellten Ausführungsbeispiel sind in dem Anschlussstück 304 eine von der Druckkammer 312 abzweigende, der Düsenöffnung 310 gegenüberliegende Bohrung 314 und eine an eine Mantelfläche des Anschlussstücks 304 angrenzend angeordnete Ausnehmung 316 vorgesehen. In dem dargestellten Ausführungsbeispiel ist die Bohrung um ca. 180° zu der Düsenöffnung 310 versetzt, in Sprührichtung stromaufwärts der Düsenöffnung 310 angeordnet. Die Ausnehmung 316 bildet eine Messkammer, welche gegenüber der Umgebung durch eine schematisch dargestellte, verformbare Membran 36 abgeschlossen ist. In dem dargestellten Ausführungsbeispiel ist die Membran 36 an einer Haltevorrichtung 4 angeordnet, und mittels der Haltevorrichtung 4 in der Ausnehmung 316 gelagert. Die Messkammer ist mit der Druckkammer 312 über die Bohrung 314 kommuniziert, sodass eine Druckerhöhung in der Druckkammer eine Druckerhöhung in der Messkammer bewirkt.

Bei einer Betätigung der Inhalationsvorrichtung 1 zum Ausbringen eines Sprühstrahls kommt es aufgrund des von dem Mediumcontainer 20 in die Druckkammer 312 abgegebenen Mediums zu einem Druckanstieg in der Druckkammer 312 und damit auch in der mit der Druckkammer 312 über die Bohrung 314 kommunizierten Messkammer 316. Der Druckanstieg bewirkt eine Verformung der Membran 36 wie schematisch durch gestrichelte Linien dargestellt. Aufgrund der Verformung der Membran 36 wird der Taster 34 betätigt. Dadurch wird ein Austragvorgang unmittelbar, basierend auf dem mit dem Austragvorgang einhergehenden Druckanstieg in der Druckkammer 312 erfasst. Eine Verarbeitung der Betätigung des Tasters 34 erfolgt mittels einer in den Figuren nicht dargestellten, beispielsweise durch den Verschlussdeckel 32 eingehausten Elektronik. Die Elektronik ist dabei in einer Ausgestaltung entsprechend DE 10 2010 024 912 A1 ausgebildet, auf welche hiermit vollumfänglich Bezug genommen wird.

An dem Verschlussdeckel 32 ist in dem dargestellten Ausführungsbeispiel weiter eine Anzeigevorrichtung 5 vorgesehen, mittels welcher die Zahl der erfassten Austragvorgänge und/oder die Zahl der verbleibenden Dosen in dem Mediumcontainer 20 anzeigbar sind.

In dem dargestellten Ausführungsbeispiel sind die Bohrung 314 und die Messkammer - bei vorschriftsgemäßem Gebrauch und einer aufrechten Orientierung der Inhalationsvorrichtung1 wie in Fig. 1 dargestellt - oberhalb der Düsenöffnung 310 und oberhalb einer Auslassöffnung des Auslassstutzens 22 angeordnet. Durch diese Anordnung der Bohrung 314 und der Messkammer ist eine Erfassung des Druckanstiegs aufgrund der Menge des ausgetragenen Mediums möglich. Eine mit der Abgabe des Mediums in die Druckkammer 312 verursachte Impulskraft wirkt dagegen nicht oder nur unwesentlich auf die Membran 36. Die dargestellte Anordnung der Messkammer 316, des Tasters 34 und der Membran 36 ist somit vorteilhaft, da durch die Anordnung eine sichere Unterscheidung eines vollständigen Sprühs von einem nicht vollständigen Sprüh möglich ist. Andere Gestaltungen, bei welchen ein Taster mittels einer reversibel verformbaren Membran betätigt wird, sind jedoch denkbar.

## Patentansprüche

1. Gehäuse für eine Containereinheit (2) einer Inhalationsvorrichtung (1) zur oralen Verabreichung eines pharmazeutischen Mediums, wobei die Containereinheit (2) einen Mediumcontainer (20) und einen relativ zu dem Mediumcontainer (20) zum Zwecke eines Austragvorgangs verlagerbaren Auslassstutzen (22) aufweist, das Gehäuse (3) umfassend
- ein Grundgehäuse (30) mit
∘ einem ersten, ein Mundstück bildenden Ende (300),
∘ einem zweiten, zu dem ersten Ende (300) L-förmig abgewinkelten, einen Lufteinlass bildenden Ende (302), und
∘ einem Anschlussstück (304),
wobei das Anschlussstück (304) einen Kanal (306) mit einer Anschlussöffnung (308) zur Aufnahme des Auslassstutzens (22) der Containereinheit (2), einer in Richtung des ersten Endes (300) mündenden Düsenöffnung (310) und einer zwischen der Anschlussöffnung (308) und der Düsenöffnung (310) angeordnete Druckkammer (312) aufweist, und
- einen Taster (34) zur Erfassung eines Austragvorgangs,
wobei eine in Abhängigkeit eines Drucks in der Druckkammer (312) reversibel verformbare Membran (36) vorgesehen ist, welche mit dem Taster (34) für dessen Betätigung zusammenwirkt,
**dadurch gekennzeichnet, dass**
dass eine mit der Druckkammer (312) kommunizierte, durch die verformbare Membran (36) gegenüber der Umgebung abgeschlossene Messkammer vorgesehen ist, so dass der Taster (34), welcher durch die sich verformende Membran (36) betätigt wird, räumlich getrennt zu der Druckkammer (312) vorgesehen ist, wobei die Messkammer (36) mit der Druckkammer (312) über eine Bohrung (314) kommuniziert ist.

2. Gehäuse nach Anspruch 1, **dadurch gekennzeichnet, dass** ein Durchmesser der Bohrung (314) zwischen ca. 120% und ca. 500% der Durchmesser der Düsenöffnung (310) beträgt.

3. Gehäuse nach Anspruch 2, **dadurch gekennzeichnet, dass** die Bohrung (314) quer, vorzugsweise im Wesentlichen senkrecht zu einer Sprührichtung der Containereinheit (2) in Sprührichtung stromaufwärts der Düsenöffnung (310) von der Druckkammer (312) abzweigt.

4. Gehäuse nach einem der vorstehenden Ansprüche, **dadurch gekennzeichnet, dass** in dem Anschlussstück (304) eine Ausnehmung (316) zur Bildung der Messkammer vorgesehen ist.

5. Gehäuse nach Anspruch 3 oder 4, **dadurch gekennzeichnet, dass** in die Ausnehmung (316) eine die Membran (36) aufweisende Haltevorrichtung (4) eingesetzt ist, wobei vorzugsweise die Membran (36) und die Haltevorrichtung (4) als einstückiges Bauteil gefertigt sind.

6. Gehäuse nach Anspruch 5, **dadurch gekennzeichnet, dass** der Taster (34) an der Haltevorrichtung (4) gelagert ist.

7. Gehäuse nach einem der Ansprüche 1 bis 6, **dadurch gekennzeichnet, dass** der Taster (34) ausgelegt und/oder angeordnet ist, um zu erfassen, wie stark und/oder wie schnell eine Betätigung erfolgte.

8. Gehäuse nach einem der Ansprüche 1 bis 7, **dadurch gekennzeichnet, dass** das Gehäuse (3) mehrteilig ausgebildet ist, umfassend das Grundgehäuse (30) und einen an dem Grundgehäuse (30) anbringbaren, elektronische Komponenten einer Zähleinheit einhausenden Verschlussdeckel (32).

9. Inhalationsvorrichtung zur oralen Verabreichung eines pharmazeutischen Mediums umfassend
- eine Containereinheit (2) mit einem Mediumcontainer (20) und einem Auslassstutzen (22) und
- ein Gehäuse (3) nach einem der Ansprüche 1 bis 8.

## Claims

1. Housing for a container unit (2) of an inhalation device (1) for orally administering a pharmaceutical medium, wherein the container unit (2) has a medium container (20) and an outlet stub (22) that is displaceable relative to the medium container (20) for the purposes of a delivery operation, the housing (3) comprising
- a main housing (30) having
∘ a first end (300) that forms a mouthpiece,
∘ a second end (302) that is angled in an L-shaped manner with respect to the first end (300) and forms an air inlet, and
∘ an attachment piece (304),
wherein the attachment piece (304) has a duct (306) with an attachment opening (308) for receiving the outlet stub (22) of the container unit (2), a nozzle opening (310) that leads out in the direction of the first end (300), and a pressure chamber (312) arranged between the attachment opening (308) and the nozzle opening (310), and
- a sensing device (34) for recording a delivery operation,
wherein a diaphragm (36) that is reversibly deformable depending on a pressure in the pressure chamber (312) is provided, said diaphragm (36) cooperating with the sensing device (34) for the actuation thereof,
**characterized in that**
a measuring chamber that is in communication with the pressure chamber (312) and is closed off from the environment by the deformable diaphragm (36) is provided, such that the sensing device (34), which is actuated by the deforming membrane (36), is provided in a spatially separate manner from the pressure chamber (312), wherein the measuring chamber communicates with the pressure chamber (312) via a hole (314).

2. Housing according to Claim 1, **characterized in that** a diameter of the hole (314) is between about 120% and about 500% of the diameter of the nozzle opening (310).

3. Housing according to Claim 2, **characterized in that** the hole (314) branches off from the pressure chamber (312) transversely, preferably substantially perpendicularly to a spraying direction of the container unit (2), downstream of the nozzle opening (310) in the spraying direction.

4. Housing according to one of the preceding claims, **characterized in that** a recess (316) for forming the measuring chamber is provided in the attachment piece (304).

5. Housing according to Claim 3 or 4, **characterized in that** a holding device (4) having the diaphragm (36) is inserted in the recess (316), wherein preferably the diaphragm (36) and the holding device (4) are manufactured as a one-piece component.

6. Housing according to Claim 5, **characterized in that** the sensing device (34) is mounted on the holding device (4).

7. Housing according to one of Claims 1 to 6, **characterized in that** the sensing device (34) is designed and/or arranged so as to record how strongly and/or how quickly an actuation has taken place.

8. Housing according to one of Claims 1 to 7, **characterized in that** the housing (3) is configured in a multipart manner, comprising the main housing (30) and a cover (32) that is fittable on the main housing (30) and encloses electronic components of a counting unit.

9. Inhalation device for orally administering a pharmaceutical medium, comprising
- a container unit (2) having a medium container (20) and an outlet stub (22), and
- a housing (3) according to one of Claims 1 to 8 .

## Revendications

1. Boîtier pour une unité de contenant (2) d'un dispositif d'inhalation (1) pour l'administration orale d'une substance pharmaceutique, l'unité de contenant (2) présentant un contenant de substance (20) et une tubulure de sortie (22) pouvant être déplacée par rapport au contenant de substance (20) pour permettre une opération de décharge, le boîtier (3) comprenant :
- un boîtier de base (30) avec
- une première extrémité (300) formant une embouchure,
- une deuxième extrémité (302) formant une entrée d'air, coudée en forme de L par rapport à la première extrémité (300), et
- un raccord (304),
le raccord (304) présentant un canal (306) avec une ouverture de raccordement (308) pour recevoir la tubulure de sortie (22) de l'unité de contenant (2), une ouverture de buse (310) débouchant dans la direction de la première extrémité (300) et une chambre de pression (312) disposée entre l'ouverture de raccordement (308) et l'ouverture de buse (310), et
- un capteur (34) pour détecter une opération de décharge,
une membrane (36) déformable de manière réversible en fonction d'une pression dans la chambre de pression (312) étant prévue, laquelle coopère avec le capteur (34) en vue de son actionnement,
**caractérisé en ce**
**qu'**il est prévu une chambre de mesure fermée par rapport à l'environnement par la membrane déformable (36), communiquant avec la chambre de pression (312), de telle sorte que le capteur (34), qui est actionné par la membrane (36) se déformant, soit prévu de manière séparé spatialement de la chambre de pression (312), la chambre de mesure communiquant avec la chambre de pression (312) par le biais d'un alésage (314).

2. Boîtier selon la revendication 1, **caractérisé en ce qu'**un diamètre de l'alésage (314) est compris entre environ 120 % et environ 500 % du diamètre de l'ouverture de buse (310).

3. Boîtier selon la revendication 2, **caractérisé en ce que** l'alésage (314) part de la chambre de pression (312) transversalement, de préférence essentiellement perpendiculairement, à une direction de pulvérisation de l'unité de contenant (2) dans la direction de pulvérisation en amont de l'ouverture de buse (310).

4. Boîtier selon l'une quelconque des revendications précédentes, **caractérisé en ce que** dans le raccord (304) est prévu un évidement (316) pour former la chambre de mesure.

5. Boîtier selon la revendication 3 ou 4, **caractérisé en ce que** dans l'évidement (316) est inséré un dispositif de retenue (4) présentant la membrane (36), la membrane (36) et le dispositif de retenue (4) étant de préférence fabriqués sous forme de composant d'une seule pièce.

6. Boîtier selon la revendication 5, **caractérisé en ce que** le capteur (34) est supporté sur le dispositif de retenue (4).

7. Boîtier selon l'une quelconque des revendications 1 à 6, **caractérisé en ce que** le capteur (34) est conçu et/ou disposé de manière à détecter avec quelle force et/ou quelle rapidité un actionnement a été effectué.

8. Boîtier selon l'une quelconque des revendications 1 à 7, **caractérisé en ce que** le boîtier (3) est réalisé en plusieurs parties, comprenant le boîtier de base (30) et un couvercle de fermeture (32) pouvant être monté sur le boîtier de base (30), recevant des composants électroniques d'une unité de comptage.

9. Dispositif d'inhalation pour l'administration orale d'une substance pharmaceutique, comprenant :
- une unité de contenant (2) avec un contenant de substance (20) et une tubulure de sortie (22) et
- un boîtier (3) selon l'une quelconque des revendications 1 à 8.
